# EUROPÄISCHE PATENTSCHRIFT

(11) **EP 1 250 913 B1**
(45) Veröffentlichungstag und Bekanntmachung des Hinweises auf die Patenterteilung: **12.04.2006**
(21) Anmeldenummer: 99961364.9
(22) Anmeldetag: 24.12.1999
(51) Int. Cl.: A61Q 5/10, A61K 8/66, A61K 8/49, A61K 8/31, A61K 8/33

(54) **NACHSCHÄUMENDE EINKOMPONENTEN-OXIDATIONSHAARFÄRBEZUBEREITUNG**
ONE-PACK TYPE POST-FOAMABLE OXIDATION HAIR-DYE COMPOSITIONS
COMPOSITIONS DE COLORANT CAPILLAIRE D'OXYDATION MOUSSANT DE TYPE MONOCOMPOSANT

(43) Veröffentlichungstag der Anmeldung: 23.10.2002
(73) Patentinhaber: SCHWARZKOPF & HENKEL K.K., Shinagawa-ku Tokyo (JP)
(72) Erfinder: TSUJINO, Yoshio, Kobe 658-0003 (JP); AOKI, Masahiro., Suminoe-ku, Osaka 559-0006 (JP)
(74) Vertreter: Stevermann, Birgit
(86) Internationale Anmeldenummer: PCT/JP1999/007273
(87) Internationale Veröffentlichungsnummer: WO 2001/047487

(56) Entgegenhaltungen:
- EP-A- 0 875 241
- EP-A1- 0 310 675
- EP-A1- 0 716 846
- EP-A2- 0 875 241
- WO-A1-99/15137
- JP-A- 9 077 629
- JP-A- 9 077 630
- JP-A- 10 316 532
- US-A- 5 902 225

## Beschreibung

### Technisches Gebiet

Gegenstand der vorliegenden Erfindung sind Zusammensetzungen von Oxidationshaarfarben, die als einkomponentiges Mittel dargereicht werden (Einkomponentenhaarfarbe). Insbesondere betrifft die Erfindung Einkomponenten-Zusammensetzungen nachschäumender Oxidationshaarfarben, die bei ihrer Anwendung ein angenehmes Gefühl vermitteln, einfach anzuwenden sind und eine vorzügliche Haarfärbewirkung zeigen.

### Stand der Technik

Herkömmliche Oxidationshaarfarben bestehen aus einem einen Oxidationsfarbstoff enthaltenden ersten Mittel und einem ein Oxidationsmittel enthaltenden zweiten Mittel, die direkt vor der Anwendung miteinander vermischt werden. Die haarfärbende Wirkung beruht auf der Oxidationspolymerisation der Oxidationsfarbstoffe. Die hier zum Einsatz kommenden Oxidationsmittel können aufgrund ihrer starken Oxidationswirkung zu einer beträchtlichen Schädigung des Haares führen. Die Erfinder haben daher ihr Augenmerk auf die milde Oxidationswirkung von Enzymen gerichtet und eine Oxidationshaarfarbenzusammensetzung (JP 63-246313 A) vorgeschlagen, welche als enzymatischen Akzeptor eine Zweielektronentransfer-Oxidase und als Donator ein entsprechendes Substrat enthält und die enzymatische Oxidation von Oxidationsfarbstoffen zur Haarfärbung nutzt. Die Anwendung dieser Erfindung ermöglicht, wenn für einen Abschluß der Zusammensetzung gegen Sauerstoff gesorgt wird, eine Ausführung von Oxidationshaarfarben als Einkomponentenmittel, da die Oxidation erst bei Kontakt mit Sauerstoff eintritt.

Durch weitere Studien gelang es den Erfindern, durch die Auswahl einer definierten Kombination von Inhaltsstoffen die Stabilität der Uricase, welche eine der in den durch die JP 63-246313 offenbarten Haarfärbemitteln eingesetzten Oxidasen ist, zu verbessern. Dadurch wurden Haarfärbemittel mit einer besseren praktischen Nutzbarkeit erhalten (JP 8-217652 A).

Bei diesen Einkomponenten-Oxidationshaarfarben, in denen Uricase verwendet wird, stellte jedoch die schlechte Löslichkeit des Uricase-Substrats Harnsäure ein Problem dar. Zur Abstellung dieses Mangels haben die Erfinder daher eine Solubilisierungstechnik für Harnsäure vorgeschlagen (JP 10-298027 A). Diese Erfindung ermöglicht intensivere Färbeergebnisse bei geringerer Farbstoffkonzentration, da Harnsäure hier in einem solubilisierten, und nicht wie zuvor in einem dispergierten, Zustand vorliegt.

Diese Einkomponenten-Oxidationshaarfarben, in denen diese Solubilisierungstechnik zum Einsatz kommt, bilden jedoch im Vergleich zu den bekannten Zweikomponenten-Oxidationshaarfarben weniger Wasserstoffperoxid, weshalb zwar der schädigende Angriff auf das Haar milder bleibt, aber unvorteilhafterweise auch die Färbewirkung schwächer wird.

### Offenbarung der Erfindung

Durch intensive Studien haben die Erfinder gefunden, daß die Färbewirkung, die bisher unbefriedigend war, durch die Zumischung von Nachschäumungsmitteln verbessert wird. Es gibt zwar bereits zahlreiche Anmeldungen zu nachschäumenden Zusammensetzungen (siehe JP 6-93126 A, JP 7-163861 A, JP 9-077629 A, JP 9-077630 A und JP 10-316532 A), da Oxidationshaarfarben jedoch üblicherweise als Mittel vorliegen, die aus zwei Komponenten bestehen, welche direkt vor der Anwendung miteinander vermischt werden müssen, können diese Zweikomponenten-Zusammensetzungen nur schwer nachschäumend formuliert werden. Es ist nun mit dieser Einkomponenten-Zusammensetzung erstmals möglich, eine Oxidationshaarfarbe als nachschäumendes Mittel zu formulieren.

Nachschäumen meint hier, daß die Zusammensetzungen direkt nach ihrer Applikation auf das Haar als Gel vorliegen, dann jedoch beim Verteilen im Haar, beispielsweise mittels einer Bürste, aufschäumen. Die erfindungsgemäßen Oxidationshaarfarben vermitteln daher ein angenehmes Gefühl bei der Anwendung und sind einfach anzuwenden, da bei der Anwendung nachvollzogen werden kann, wo die Zusammensetzungen aufgetragen wurden, und die Zusammensetzungen außerdem nicht vom Haar ablaufen.

### Detaillierte Beschreibung der Erfindung

Bei der in erfindungsgemäßen nachschäumenden Einkomponenten-Oxidationshaarfarbenzusammensetzungen als Inhaltsstoff eingesetzten Uricase handelt es sich um eine Oxidase, die auch unter dem Namen Harnsäure-Oxidase bekannt ist. Uricase ist eine Zweielektronentransfer-Oxidase, die, mit Ausnahme von Primaten, in der Leber, den Nieren und der Milz von Säugetieren in größeren Mengen vorzufinden ist. Die Uricase-Konzentration in erfindungsgemäßen Zusammensetzungen liegt bei 100 bis 50.000 IU/100 g, vorzugsweise jedoch in einem Bereich von 1.000 bis 30.000 IU/100 g. Bei einer Uricase-Konzentration unter 100 IU/1 00 g bleibt die Färbewirkung ungenügend, während Konzentrationen über 50.000 IU/100 g in Kostenhinsicht ungünstig sind, da sie zu keiner dem höheren Enzymeinsatz entsprechenden Verbesserung der Färbewirkung führen. Die hier verwendete Einheit für die Enzymaktivität "IU" meint die internationale Einheit, wobei die einer Enzymaktivität von 1 IU entsprechende Enzymmenge der Menge an Enzym entspricht, mit der bei 25 °C und einem pH-Wert von 8,5 in 1 Minute 1 µmol Harnsäure umgesetzt wird.

Ein weiterer obligatorischer Inhaltsstoff erfindungsgemäßer nachschäumender Einkomponenten-Oxidationshaarfarbenzusammensetzungen ist das Uricase-Substrat Harnsäure. Durch die Umsetzung des als Donator fungierenden Substrates Harnsäure mit Uricase unter geeigneten Bedingungen während der Anwendung der erfindungsgemäßen Oxidationshaarfarbenzusammensetzungen werden aufgrund dieser enzymatischen Oxidationsreaktion die Oxidationsfarbstoffe der Zusammensetzungen im Haar polymerisiert, wodurch Farbstoffe gebildet werden und das Haar somit gefärbt wird. Der Begriff "Harnsäure" umfaßt in dieser Erfindung auch die Salze der Harnsäure. Der Gehalt an Harnsäure in diesem Sinne in erfindungsgemäßen Oxidationshaarfarbenzusammensetzungen liegt vorzugsweise bei 0,1 bis 5,0 Gewichts-%.

Wie im Falle des Enzyms bleibt bei einem geringeren Gehalt an der als Donator fungierenden Harnsäure das Färbeergebnis unzureichend, während höhere Harnsäureanteile sich als unwirtschaftlich erweisen.

Nachschäumungsmittel bilden eine weitere Gruppe obligatorischer Inhaltsstoffe in erfindungsgemäßen nachschäumenden Einkomponenten-Oxidationshaarfarbenzusammensetzungen. Der Siedepunkt der in der Erfindung verwendeten Nachschäumungsmittel liegt bei höchstens 40 °C, als Beispiele für derartige Nachschäumungsmittel werden aliphatische Kohlenwasserstoffe wie etwa Propan, Isobutan, n-Butan, Pentan und Isopentan sowie Dimethylether und Divinylether genannt. Als Anteil dieser Nachschäumungsmittel in den Oxidationshaarfarbenzusammensetzungen sind 0,5 bis 3,0 Gewichts-% angemessen. Bei Anteilen unter 0,1 Gewichts-% bleibt die Nachschäumwirkung unzureichend, während umgekehrt Anteile über 10,0 Gewichts-% zu einem sofortigen Aufschäumen führen und daher für ein gutes nachschäumendes Verhalten unangemessen sind.

Der pH-Wert erfindungsgemäßer nachschäumender Einkomponenten-Oxidationshaarfarbenzusammensetzungen wird in einem Bereich von 7,5 bis 9,5 eingestellt. Als Beispiele für Mittel zum Einstellen des pH-Wertes (pH-Regulatoren) werden alkalische Amine auf Aminbasis, etwa Monoethanolamin (MEA), Diethanolamin (DEA), Triethanolamin (TEA), 2-Amino-2-methyl-1-propanol (AMP), 2-Amino-2-methyl-1,3-propandiol (AMPD), Monoisopropanolamin (MIPA) und Tetrakis(2-hydroxyisopropyl)-ethylendiamin (TE) genannt. Diese pH-Regulatoren können sowohl einzeln als auch in Form von Kombinationen eingesetzt werden, außerdem können Ammoniak und andere anorganische alkalische Mittel wie etwa Natriumhydroxid und Kaliumhydroxid als pH-Regulatoren verwendet werden.

Die in erfindungsgemäßen Zusammensetzungen verwendeten Oxidationsfarbstoffe sind hinsichtlich Art und Dosierung keinen besonderen Einschränkungen unterworfen und werden aus den bekannten Oxidationsfarbstoffen ausgewählt. Beispielsweise können, in jeweils geeigneten Mengen, die in den "Japanese Standards of Quasi-Drug Ingredients" (verlegt von The Yakuji Nippo Ltd., redigiert von der Society of Japanese Pharmacopoeia unter Anleitung und Überwachung der Pharmaceutical and Cosmetics Division, Pharmaceutical Affairs Bureau, Ministry of Health and Welfare, Juni 1991) aufgeführten Oxidationsfarbstoffe 5-Amino-o-cresol, 3,3'-Iminodiphenol, 2,4-Diaminophenolhydrochlorid, 2,5-Diaminotoluolhydrochlorid, *p-*Phenylendiaminhydrochlorid, N-Phenyl-*p*-phenylendiaminhydrochlorid, *m-*Phenylendiaminhydrochlorid, *o*-Aminophenol, Brenzcatechin, N-Phenyl-*p-*phenylendiaminacetat, 2,6-Diaminopyridin, 1,5-Dihydroxynaphthalin, Diphenylamin, 2,5-Diaminotoluol, 3,4-Diaminotoluol, alpha-Naphthol, *p*-Aminophenol, *p*-Phenylendiamin, *p*-(Methylamino)phenol, Hydrochinon, Pyrogallol, N-Phenyl-*p-*phenylendiamin, Phloroglucin, *m*-Aminophenol, *m*-Phenylendiamin, 5-Amino-*o-*cresolsulfat, *o*-Aminophenolsulfat, *o*-Chlor-*p*-phenylendiaminsulfat, 4,4'-Diaminodiphenylaminsulfat, 2,4-Diaminophenolsulfat, 2,5-Diaminotoluolsulfat, *p-*Aminophenolsulfat, *p*-Phenylendiaminsulfat, *p*-Methylaminophenolsulfat, *m*-Aminophenolsulfat, *m*-Phenylendiaminsulfat, 2,4-Diaminophenoxyethanolhydrochlorid und 5-(2-hydroxyethylamino)-2-methylphenol verwendet werden. Außerdem kann auch das in den oben zitierten Normen nicht aufgeführte 2,2'-[(4-aminophenyl)imino]bisethanol in geeigneter Dosierung verwendet werden.

Desweiteren umfaßt der Begriff der Oxidationsfarbstoffe im weiteren Sinne dieser Anmeldung auch direktziehende Farbstoffe, die, wie etwa 2-Amino-4-nitrophenol, 2-Amino-5-nitrophenol, 1-Amino-4-methylaminoanthrachinon, Nitro-*p*-phenylendiaminhydrochlorid, 1,4-Diaminoanthrachinon, Nitro-*p*-phenylendiamin, Picraminsäure, Natriumpicramat, 2-Amino-5-nitrophenolsulfat, Resorcin, Nitro-*p*-phenylendiaminsulfat, *p*-Nitro-*o*-phenylendiaminsulfat, *p*-Nitro-*m*-phenylendiaminsulfat und dergleichen üblicherweise häufig in Kombination mit Oxidationsfarbstoffen verwendet werden. Von diesen Oxidationsfarbstoffen werden in erfindungsgemäßen Zusammensetzungen vorzugsweise *p*-Phenylendiamin und dessen Salze, *p*-Aminophenol, *o*-Aminophenol, m-Aminophenol, *p*-Nitro-*o*-phenylendiamin, 2,6-Diaminopyridin, Resorcin, *o*-Aminophenol und *m*-Phenylendiaminhydrochlorid verwendet. Die vorgenannten Oxidationsfarbstoffe können in der Erfindung sowohl einzeln als auch in Form von Kombinationen verwendet werden, die mindestens zwei der vorgenannten Oxidationsfarbstoffe enthalten.

Die erfindungsgemäßen nachschäumenden Einkomponenten-Oxidationshaarfarbenzusammensetzungen können durch den Einsatz von Nachschäumungsmitteln auch auf bereits bekannte Oxidationshaarfarben angewendet werden, in denen Zweielektronentransfer-Oxidase und Substrate verwendet werden. Als Beispiele für derartige Oxidationshaarfarben, auf die die Erfindung angewendet werden kann, werden die aus der WO 98/55083, der WO 99/17720, der WO 99/17721, der WO 99/17722, der WO 99/17723, der WO 99/17724, der WO 99/17725, der WO 99/17726, der WO 99/17727, der WO 99/17728, der WO 99/17729, der WO 99/17730, der WO 99/17731, der WO 99/17732, der WO 99/17733, der JP-A 63-246313, der JP-A 8-217652, der JP-A 10-298027 und der JP-A 11-12153 bekannten Oxidationshaarfarben genannt.

### Bevorzugte Ausführungsformen der Erfindung

Im folgenden werden die erfindungsgemäßen Zusammensetzungen nachschäumender Einkomponenten-Oxidationshaarfarben durch Prüfungs-, Ausführungs- und

Vergleichsbeispiele illustriert.

### Prüfungsbeispiele

Unter Verwendung der in Tabelle 1 genannten Inhaltsstoffe und von Isopentan als Nachschäumungsmittel in verschiedenen Anteilen wurden Zusammensetzungen von nachschäumenden Einkomponenten-Oxidationshaarfarben zubereitet. Zum Vergleich wurde eine Oxidationshaarfarbenzusammensetzung ohne Isopentan mit ansonsten jedoch gleicher Rezeptur zubereitet.

**Tabelle 1: Prüfungsbeispiele**

| Inhaltsstoffe | Nr. 1 | Nr. 2 | Nr. 3 | Nr. 4 | Nr. 5 | Vergl.*) |
|---|---|---|---|---|---|---|
| *p*-Phenylendiamin | 0,60 | 0,60 | 0,60 | 0,60 | 0,60 | 0,60 |
| *p*-Methylaminophenolsulfat | 0,30 | 0,30 | 0,30 | 0,30 | 0,30 | 0,30 |
| 2,4-Diaminophenoxyethanol- | | | | | | |
| hydrochlorid | 0,05 | 0,05 | 0,05 | 0,05 | 0,05 | 0,05 |
| *p*-Aminophenol | 0,10 | 0,10 | 0,10 | 0,10 | 0,10 | 0,10 |
| 5-Amino-*o*-cresol | 0,05 | 0,05 | 0,05 | 0,05 | 0,05 | 0,05 |
| Resorcin | 0,50 | 0,50 | 0,50 | 0,50 | 0,50 | 0,50 |
| N-Acetyl-L-Cystein | 0,08 | 0,08 | 0,08 | 0,08 | 0,08 | 0,08 |
| Alkylacrylat-Alkylmethacrylat- | | | | | | |
| Polyoxy-ethylenester-Copolymer | | | | | | |
| Emulsion (30%) | 2,50 | 2,50 | 2,50 | 2,50 | 2,50 | 2,50 |
| Sorbit | 3,00 | 3,00 | 3,00 | 3,00 | 3,00 | 3,00 |
| Polyoxyethylenmethylglucosid | 1,00 | 1,00 | 1,00 | 1,00 | 1,00 | 1,00 |
| Polypeptid-Kokosfettsäure-Kalium | 1,00 | 1,00 | 1,00 | 1,00 | 1,00 | 1,00 |
| Monoethanolamin | ad pH 9,2 | | | | | |
| Harnsäure | 1,00 | 1,00 | 1,00 | 1,00 | 1,00 | 1,00 |
| Uricase (20 IU/mg) | 1,00 | 1,00 | 1,00 | 1,00 | 1,00 | 1,00 |
| Isopentan | 0,50 | 1,00 | 1,50 | 2,00 | 3,00 | 0,00 |
| Aqua purificata | ad 100 | | | | | |

| | | | | | | |
|---|---|---|---|---|---|---|
| *) zum Vergleich | | | | | | |

Mit den Zusammensetzungen nachschäumender Einkomponenten-Oxidationshaarfarben der Prüfungsbeispiele 1 bis 5 und der Vergleichszusammensetzung wurde eine Haarfärbeprüfung durchgeführt. Hierzu wurden Ziegenhaarsträhnen mit den Zusammensetzungen bei einem Flottenverhältnis von 1 zu 2 (Volumen Ziegenhaarsträhnen zu Volumen der Zusammensetzungen) 30 min bei 30 °C gefärbt und anschließend gewaschen und getrocknet. Die Haarfärbewirkung der Zusammensetzungen wurde durch Begutachtung der gefärbten getrockneten Haarsträhnen mit bloßem Auge gemäß folgender Kriterien bewertet. Die Ergebnisse sind in Tabelle 2 zusammengestellt.
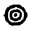 : intensive Färbung
○: vergleichsweise gute Färbung (gleicher Färbegrad wie bei Färbung mit einem Vergleichsbeispiel)
Δ: schwache Färbung
X: sehr schwache Färbung

**Tabelle 2**

| | Prüfungsbeispiel Nr. | | | VB*) | | |
|---|---|---|---|---|---|---|
| | 1 | 2 | 3 | 4 | 5 | 1 |
| Haarfarbewirkung | ⊙ | ⊙ | ⊙ | ⊙ | ⊙ | ○ |
| | *) Vergleichsbeispiel Nr. | | | | | |

Wie von den Ergebnissen in Tabelle 2 abzulesen ist, zeigen die erfindungsgemäßen Zusammensetzungen nachschäumender Einkomponenten-Oxidationshaarfarben infolge ihres nachschäumenden Verhaltens eine verbesserte Haarfärbewirkung.

### Ausführungsbeispiel 1

In einem üblichen Verfahren wurde eine erfindungsgemäße Haarfarbenzusammensetzung mit folgender Rezeptur zubereitet:

| **Inhaltsstoffe** | **Anteil (in Gew.-%)** |
|---|---|
| *p*-Phenylendiamin | 0,5 |
| 2,4-Diaminophenoxyethanolhydrochlorid | 0,05 |
| 2-[[4-(Dimethylamino)phenyl]azo]-1,3-dimethylimidazoliumchlorid | 0,1 |
| (1-Methyl-1-phenyl)-2-(1-methin-4N-methyl- pyridinium)hydrazinchlorid | 0,05 |
| 4-Aminophenylazo-2N-methyl-5N-methylimidazol- yliumchlorid | 0,05 |
| L-Cystein | 0,2 |
| Alkylacrylat-Alkylmethacrylat-Polyoxy-ethylenester-Copolymer Emulsion (30%) | 3,5 |
| Dekaglycerylmonomyristat | 1,0 |
| Monoisopropanolamin | ad pH 9,0 |
| Harnsäure | 1,0 |
| Uricase (20 IU/mg) | 1,0 |
| Isopentan | 2,0 |
| Aqua purificata | ad 100 |
| Summe | 100,0 |

### Ausführungsbeispiel 2

In einem üblichen Verfahren wurde eine erfindungsgemäße Haarfarbenzusammensetzung mit folgender Rezeptur zubereitet:

| **Inhaltsstoffe** | **Anteil (in Gew.-%)** |
|---|---|
| *p*-Phenylendiamin | 0,6 |
| m-Phenylendiaminhydrochlorid | 0,1 |
| Thioglykolsäure | 0,2 |
| Xanthangummi | 2,0 |
| Polyoxyethylen(4,2)Laurylether | 3,0 |
| Kaliumhydroxid | pH ad 9,0 |
| Harnsäure | 1,0 |
| Uricase (20 IU/mg) | 1,0 |
| Isopentan | 2,0 |
| Aqua purificata | ad 100 |
| Summe | 100,0 |

### Ausführungsbeispiel 3

In einem üblichen Verfahren wurde eine erfindungsgemäße Haarfarbenzusammensetzung mit folgender Rezeptur zubereitet:

| **Inhaltsstoffe** | **Anteil (in Gewichts-%)** |
|---|---|
| *p*-Phenylendiamin | 0,3 |
| 2-Amino-3-hydroxypyridin | 0,3 |
| N-Acetyl-L-cystein | 0,2 |
| Ethanol | 20,0 |
| Hydroxyethylcellulose | 2,0 |
| C₈₋₁₀-Alkylglucosid | 5,0 |
| Monoethanolamin | ad pH 9,0 |
| Harnsäure | 1,0 |
| Uricase (20 IU/mg) | 1,0 |
| Isopentan | 2,0 |
| Aqua purificata | ad 100 |
| Summe | 100,0 |

### Ausführungsbeispiel 4

In einem üblichen Verfahren wurde eine erfindungsgemäße Haarfarbenzusammensetzung mit folgender Rezeptur zubereitet:

| **Inhaltsstoffe** | **Anteil (in Gewichts-%)** |
|---|---|
| *p*-Phenylendiamin | 0,5 |
| 1,3-Dihydroxybenzol | 0,3 |
| N-Acetyl-L-cystein | 0,2 |
| Propylenglykolmonomethylether | 20,0 |
| Hydroxyethylcellulose | 2,0 |
| C₈₋₁₀-Alkylglucosid | 5,0 |
| Monoethanolamin | ad pH 9,0 |
| Harnsäure | 1,0 |
| Uricase (20 IU/mg) | 1,0 |
| Isopentan | 2,0 |
| Aqua purificata | ad 100 |
| Summe | 100,0 |

### Industrielles Anwendungsgebiet

Wie oben näher erläutert, liefert die Erfindung nachschäumende Einkomponenten-Oxidationshaarfarben- Zusammensetzungen, die aufgrund ihres nachschäumenden Verhaltens eine verbesserte Haarfärbewirkung zeigen und sich zudem durch ein angenehmes Gefühl bei der Anwendung und durch einfache Anwendbarkeit auszeichnen. Die erfindungsgemäßen nachschäumenden Einkomponenten-OxidationshaarfarbenZusammensetzungen lassen sich zudem auf Oxidationshaarfarben anwenden, in denen Zweielektronentransfer-Oxidase und Substrate verwendet werden.

## Patentansprüche

1. Nachschäumende Einkomponenten-Oxidationshaarfarben-Zusammensetzungen, **dadurch gekennzeichnet, dass** sie Uricase, Hamsäure, einen Oxidationsfarbstoff und 0,5 bis 3,0 Gew.-% eines Nachschäumungsmittels enthalten.

2. Zusammensetzungen nach Anspruch 1, in denen als Nachschäumungsmittel mindestens ein organisches Lösungsmittel mit einem Siedepunkt von höchstens 40 °C verwendet wird.

3. Zusammensetzungen nach Anspruch 2, in denen das organische Lösungsmittel ausgewählt ist aus der Gruppe, die Propan, Isobutan, n-Butan, Pentan, Isopentan und Dimethylether umfaßt.

4. Zusammensetzungen nach Anspruch 1, die einen oder mehrere Oxidationsfarbstoffe in Anteilen von 0,1 bis 5,0 Gewichts-% und Harnsäure in Anteilen von 0,1 bis 5,0 Gewichts-% enthalten.

5. Zusammensetzungen nach Anspruch 1, die Uricase mit einer Konzentration von 1.000 bis 30.000 IU/100 g enthalten.

## Revendications

1. Compositions de teinture par oxydation des cheveux à post-moussage et à un composant, **caractérisées en ce qu'**elles contiennent de l'uricase, de l'acide urique, un colorant d'oxydation et 0,5 à 3,0% en poids d'un agent de post-moussage.

2. Compositions selon la revendication 1 dans lesquelles on utilise comme agent de post-moussage au moins un solvant organique présentant un point d'ébullition d'au maximum 40°C.

3. Compositions selon la revendication 2 dans lesquelles le solvant organique est choisi dans le groupe comprenant le propane, l'isobutane, le n-butane, le pentane, l'isopentane et le diméthyléther.

4. Compositions selon la revendication 1 qui contiennent un ou plusieurs colorants d'oxydation en des proportions de 0,1 à 5,0% en poids et de l'acide urique en des proportions de 0,1 à 5,0% en poids.

5. Compositions selon la revendication 1 qui contiennent de l'uricase en une concentration de 1 000 à 30 000 IU/100 g.

## Claims

1. Post-foaming one-pack oxidation hair dye compositions, **characterized in that** they comprise uricase, uric acid, an oxidation dye and 0.5 to 3.0% by weight of a post-foaming agent.

2. Compositions according to Claim 1, in which the post-foaming agent used is at least one organic solvent with a boiling point of at most 40°C.

3. Compositions according to Claim 2, in which the organic solvent is chosen from the group which consists of propane, isobutane, n-butane, pentane, isopentane and dimethyl ether.

4. Compositions according to Claim 1, which comprise one or more oxidation dyes in amounts of from 0.1 to 5.0% by weight and uric acid in amounts of from 0.1 to 5.0% by weight.

5. Compositions according to Claim 1, which comprise uricase with a concentration of from 1000 to 30 000 IU/100 g.
